# EUROPEAN PATENT APPLICATION

(11) **EP 4 717 141 A1**
(43) Date of publication of application: **01.04.2026**
(21) Application number: 24202544.3
(22) Date of filing: 25.09.2024
(51) Int. Cl.: A61B 1/00, A61B 1/05, A61B 1/06

(54) **MEDICAL IMAGING DEVICE WITH ELECTRICAL NOISE MITIGATION**

(71) Applicant: Ambu A/S, 2750 Ballerup (DK)
(72) Inventor: RASK, Jesper Domino, 2300 Copenhagen S (DK); NIROUMAND, Farideh Javidi, 2450 København SV (DK)
(74) Representative: Winter, Brandl - Partnerschaft mbB

(57) **Abstract**

An intrusive medical device and a visualization system including the intrusive medical device, the intrusive medical device including a proximal end and a distal end spaced apart from the proximal end; a camera and a light source positioned at the distal end; and a cable bundle of consisting of a first and a second coaxial cable within a common shield, the cable bundle extending from the proximal end to the distal end and electrically connected to the camera and light source at the distal end with the common shield electrically connecting ground to camera and light source.

## Description

### TECHNICAL FIELD

The present disclosure relates to intrusive medical devices comprising distal end cameras, such as endoscopes and respiratory tubes, and, in particular, intrusive medical devices configured to mitigate electrical noise.

### BACKGROUND

Visualization systems including video processing apparatus electrically connected to an intrusive medical device are known and can be used for visual navigation into, and examination and diagnosis of, hollow organs and body cavities, as well as, optionally, to assist in surgery, e.g. for a targeted tissue sampling. Example intrusive medical devices include endoscopes and respiratory tubes. Endoscopes include procedure-specialized endoscopes, such as bronchoscopes, arthroscopes, cystoscopes, ureteroscopes, cholangioscopes, colonoscopes, laparoscopes, gastroscopes, and duodenoscopes. Respiratory tubes include endobronchial tubes, endotracheal tubes, tracheostomy tubes, and other tubes configured to ventilate at least a portion of the respiratory system or lungs of a patient, and may include a laryngeal mask or inflatable cuffs. A visualization system including an endoscope operable with a surgical tool is described in commonly-owned U.S. Patent No. 10,646,107. Respiratory tubes are described in commonly-owned U.S. Patents Nos. 9,486,595, 9,889,264, 10,406,309, and 10,888,679. A visualization system including a portable medical monitor having a display screen is described in commonly-owned U.S. Patents Nos. 11,266,297 and 11,328,390.

An electrosurgical instrument may be guided through a lumen of the intrusive medical device to perform medical procedures within the patient's body cavity. Known electrosurgical tools, which are operated by high voltage pulses (e.g. in a range of 2 kV to 8 kV), may generate high frequency noise in signals and interference in videos apparent to a user during a procedure. An electrosurgical tool, which is configured to perform argon plasma coagulation (APC), is an example of such electrosurgical tool. Argon plasma coagulation is an electrosurgical, monopolar procedure for superficial hemostasis, devitalization and ablation using ionized argon gas, which as an inert gas can be easily ionized. High voltage pulses result in a strong electric field (high frequency) that may be experienced as a high frequency noise on electrical conductors. Electrical noise in video signals can also be caused by high frequency clock signal (which may be, for example, 4, 12 or 24 MHz depending on the specific image sensor). This electrical noise is sometimes referred to as "hum" or "cross-clock".

Electrical noise can result in deterioration of image quality or the loss of live images for various reasons, including "freezing" of the camera module (requiring a reset) and because the communication of configuration of control data to the camera goes wrong, i.e. data is written in wrong places, or the wrong data is written, in registers of the camera.

Furthermore, known intrusive medical devices may comprise diameters greater than 3.4 mm and might have enough space to not impose size or configuration limits on the electrical conductors. However, as the intrusive medical devices become smaller in size, particularly when their distal ends are 3.4 mm or less in diameter, which is desirable to mitigate tissue damage and facilitate navigation into the patient, it is desirable to reduce the size and configuration of the electrical conductors.

Even further, it is desirable to present images on the display screen in real-time. Known medical monitors may employ image processing algorithms configured to mitigate the effects of electrical noise. For example, medical monitors might average frames of a video stream (e.g. a sequence of frames or images) or exclude frames from the video stream if the frames are defective, e.g. exhibit vertical or horizontal lines, exhibit large over or under-exposed areas, or are defective for other reasons. This may cause a physician to see a frame that is outdated, albeit by a fraction of a second, which is not ideal. If image processing algorithms are modified to present video streams at or closer to real-time, these video stream noise mitigation techniques are removed and the electrical noise in the intrusive medical devices has to be reduced or eliminated to ensure an adequate user experience.

Additionally, image processing algorithms may be modified to add features, such as navigation and tissue/object identification, which if added without removal of code may require larger, more expensive, hardware. Thus, removal of noise mitigation processing instructions may be enabled by improvements in the intrusive medical devices, resulting in overall cost reductions.

Single-use intrusive medical devices optimize workflow and reduce cost while saving patient's lives and improving patient care. They optimize workflow and reduce cost because they are always ready when needed without the traditional large-scale capital and repair budgets required for reusable intrusive medical devices. For example, a sterilization and storage facility is avoided, there is no need to maintain evidence of sterilization, and there is no need to transport intrusive medical devices from sterilization and storage facilities to the buildings where they are needed, sometimes in the middle of the night or weekends. They save patient's lives and improve patient care because they are readily available and do not pose a cross-contamination risk. This also reduces hospital re-admissions. While single-use intrusive medical devices are disposed after a single patient use (one or more procedures may be performed while the patient remains in the treatment room), the environmental impact of re-useable intrusive medical devices, due to cleaning materials, CO₂ emissions during the cleaning process, and use of disposable personal protective equipment by personnel involved in transportation and sterilization of the re-useable intrusive medical devices, can be similar to that of single-use intrusive medical devices. To further reduce environmental impact, the intrusive medical devices according to the present disclosure are primarily made of polymer materials.

For at least the foregoing reasons, it is desirable to incorporate noise mitigation features in intrusive medical devices, particularly in single-use medical devices, to improve their value and, in particular, to improve their effectiveness when electronic noise generating tools are used.

To further enhance the benefits of single-use intrusive medical devices, it is desirable to expand the applicability of the intrusive medical devices, e.g. by enabling even smaller devices.

To further enhance the benefits of single-use intrusive medical devices, it is desirable to reduce manufacturing costs.

### SUMMARY

The objectives of the present disclosure are to provide an intrusive medical device with features that eliminate or at least reduce the disadvantages of the prior art intrusive medical devices and suitably deal with the above-described problems. In particular, it is an object of the present disclosure to present an intrusive medical device that exhibits reduced electrical noise disturbance relative to prior art intrusive medical devices.

A first aspect relates to expanding the applicability of an intrusive medical device as outlined in claim 1 and a visualization system in accordance with claim 12. Advantageous embodiments are claimed in the dependent claims and/or are explained below. An advantage of the intrusive medical device of claim 1 is that electrical noise is effectively mitigated. Furthermore, the use of coaxial cables facilitates a compact construction as will be elaborated below.

In a variation of the embodiment of claim 1 the coaxial cables are untwisted. Twisted pair coaxial cables where the two coaxial cables are twisted around each other could have the potential advantage of increased mitigation of electrical noise. However, arranging the coaxial cables untwisted side-by-side allows for a cable bundle with a very low profile, which is advantageous because by having a narrow radial profile when assembled it may enable overall reductions in the diameter of the insertion cord or the distal end thereof.

In another variation of the present embodiment, the first cable shield electrically connecting the light source and configured to supply power; the first cable central conductor electrically connecting the camera and configured to transfer the video signal; the second cable shield electrically connecting the camera and configured to the supply power; and the second cable central conductor electrically connecting the camera and configured to transfer the clock signal. In other words, the first cable shield provides power to the light source; the first cable central conductor transmits the video signal from the camera; the second cable shield provides power to the camera; and the second cable central conductor transmits clock signal to the camera. This configuration separates the light source power conductor from the clock signal conductor, which may be beneficial to mitigate potential electrical noise from the light source power conductor onto the clock signal. The first cable shield; the first cable central conductor; the second cable shield and the second cable central conductor may be connected, directly or indirectly, to the light source and to the camera. The light source power conductor may be independent from the camera power conductor.

In another variation of the present embodiment, the first cable shield electrically connecting the light source and configured to supply power; the first cable central conductor electrically connecting the camera and configured to transfer the clock signal; the second cable shield electrically connecting camera and configured to supply power; the second cable central conductor electrically connecting the camera and configured to transfer the video signal. This configuration separates the light source power conductor from the video signal conductor, which may be beneficial to mitigate potential electrical noise from the light source power conductor onto the video signal.

In an example of the embodiment, the coaxial cables are micro-coaxial cables with center conductor cross-sectional area in the interval of 0.0005 mm² to 0.0055 mm², such as in the interval of 0.0008 mm² to 0.0035 mm², such as 0.0014 mm². These cross-sectional areas of the center conductor are found to be suitable for intrusive medical devices. A cross-sectional area larger than 0.0055 mm² has the advantage of robustness of the conductor and relatively low electrical resistance, but is presently not preferred due to the increased size of the cable bundle. A cross-sectional area lower than 0.0005 mm² has the advantage of facilitating a very compact construction, but the increased electrical resistance, physical fragility and generally increased cost makes this a less preferred choice.

In an example of the present embodiment, the cable bundle has a height height in the interval of 0.1 to 0.7 mm, such as in the interval of 0.2 mm to 0.6 mm, such as 0.35 mm. Cable bundle height below 0.7 mm is found advantageously for intrusive medical devices as this allows for a very compact construction with minimum outer dimension of the intrusive medical device and/or an intrusive medical device have a working channel of large dimension. Low cable bundle height is hence relevant for both small intrusive medical devices, e.g. bronchoscopes for pediatric indication, and for larger gastroscopes, as the cable bundle will not take up much space. Cable bundle height in the interval 0.2 mm to 0.6 mm is found to be a suitable compromise between compactness, cost and mitigation of electrical noise in most cases.

In an example of the present embodiment, the cable bundle has a width width in the interval of 0.2 mm to 0.9 mm, such as in the interval of 0.4 mm to 0.75 mm, such as 0.60 mm. A width of more than 0.9 mm may be acceptable in some intrusive medical devices, such as colonoscopes, but for most intrusive medical devices a cable bundle width in the interval of 0.4 mm to 0.75 mm is found is found to provide a suitable compromise between compactness, cost and mitigation of electrical noise.

In an example of the present embodiment, the cable bundle has a ratio of height to width in the interval of 0.55 to 0.75, such a 0.67.

An embodiment of the disclosure relates to an intrusive medical device, wherein the intrusive medical device comprises an endoscope including: the proximal end comprising a positioning interface having a distal end; an insertion cord connected to and extending from the distal end of the positioning interface and comprising an insertion tube, a bending section, and a tip housing, the camera positioned in the tip housing, the tubular member extending from the positioning interface through the insertion tube to the tip housing, wherein the bundle extends from the positioning interface to the tip housing.

In an example of the present embodiment the bending section comprises a wherein the bending section comprises a diameter in the interval of 1.8 mm to 6.0 mm, such as in the interval of 2.0 mm to 3 mm, such as 2.5 mm. Bending section diameter is the maximum diameter of the bending section, without any potential bending cover. A bending section diameter in the specified interval is considered a suitable compromise for e.g. bronchoscopes and rhinolaryngoscopes.

In an example of the present embodiment a ratio of the cable bundle height to bending section diameter is less than 0.2, such as in the interval of 0.04 to 0.16, e.g. approximately 0.1. A ratio above 0.2 may be acceptable for some embodiments, e.g. intrusive medical instruments without a working channel. A ratio below 0.2 is however considered advantageous as the cable bundle then takes up a relatively small percentage of the cross-section of the bending section, and a ratio in the interval of 0.04 to 0.16 is considered a suitable compromise in practice.

An aspect of the disclosure relates to a visualization system comprising: the intrusive medical device as disclosed above; and a video processing apparatus configured to communicatively connect with the intrusive medical device to receive a video stream therefrom.

One or more objects may be met by aspects of the present invention described in the following embodiments, variations and examples thereof.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail below with reference to the accompanying figures. The figures illustrate embodiments, variations and examples to facilitate the understanding of a person of ordinary skill in the art and are not to be construed as limiting the scope of the attached claim set.
FIGS. 1 and 2 are schematic illustrations of an intrusive medical device according with the detailed description;
FIG. 3 is a schematic illustration of an electrosurgical system and a visualization system including an intrusive medical device;
FIG. 4 is a perspective view of an embodiment of an intrusive medical device, exemplified by an endoscope;
FIG. 5 is a perspective view of a distal portion of the endscope of FIG. 4 with covering removed for purpose of illustration;
FIG. 6 to 10 are depictions of cross-sections of embodiments of bending sections of endoscopes including the endoscope depicted in FIGS. 4 and 5;
FIGS. 11 and 12 are plan and sectional views of another embodiment of an intrusive medical device, exemplified by a dual-lumen tube;
FIGS. 13 and 14 are perspective and plan views of embodiments of video processing apparatus operable with the intrusive medical devices of FIGS. 1-12;
FIG. 15 is a schematic sectional view of a dual coax cable,
FIG. 16 is a schematic diagram of the dual coax cable of FIG. 15 as incorporated in the illustration of FIG. 2, and

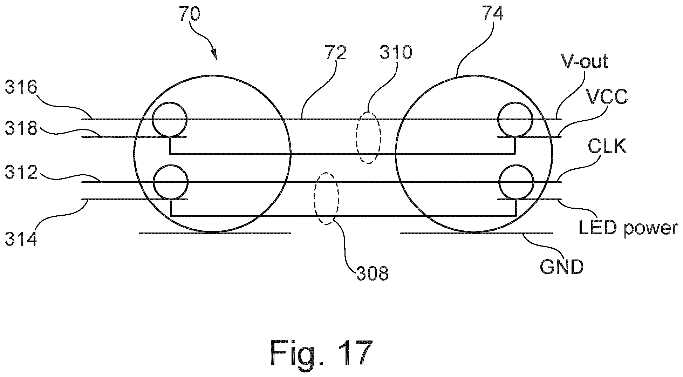

FIG. 17 is a schematic diagram of an alternative wiring of the dual coax cable of FIG. 15 as incorporated in the illustration of FIG. 2.

### DETAILED DESCRIPTION

The term "distal," as used herein, refers to a direction or position that is generally towards a target site, and the term "proximal," as used herein, refers to a direction or position that is generally away from the target site.

Unless otherwise defined, all technical and scientific terms used herein have the same meaning as commonly understood by one of ordinary skill in the art. In case of conflict, the present document, including definitions, will control. Preferred methods and materials are illustrated below, although apparatuses, methods, and materials similar or equivalent to those illustrated herein may be used in practice or testing. The materials, methods, and examples disclosed herein are illustrative only and not intended to be limiting.

As described herein, an intrusive medical device may comprise: a proximal end and a distal end spaced apart from the proximal end; a tubular member defining a lumen therein, the tubular member extending from the proximal end to the distal end; a camera and at least one light source positioned at the distal end; and a cable bundle consisting of a first and a second coaxial cable within a common electrical shield, the cable bundle extending from the proximal end to the distal end and electrically connected to the camera and light source at the distal end; the first coaxial cable comprising a first cable central conductor, a first cable insulating layer, and a first cable shield; the second coaxial cable comprising a second cable central conductor, a second cable insulating layer, and a second cable shield; one of the first or second cable shield electrically connecting to the light source and configured to supply power; another of the first or second cable shield electrically connecting to the camera and configured to supply camera power; one of the first or second cable central conductor electrically connecting to the camera and configured to transfer a video signal; another of the first or second cable central conductor electrically connecting to the camera and configured to transfer a clock signal; and the common electrical shield electrically connecting ground to camera and light source. The intrusive medical device may comprise an endoscope.

Advantageously, whether with or without a tubular member defining a lumen therein, the intrusive medical device as described above mitigates electrical noise. The electrical noise might be cross-talk and/or generated by an electro-surgical tool (EST) or other electromagnetic field. The electrical noise could also be emissions by the intrusive medical device itself which could lead to problems to live up to regulations on electromagnetic compatibility (EMC). The video signal conductor may transfer digital or analog video data. The video signal conductor may also transfer control signals in addition to video signals.

The bundle consisting of two coaxial cables may be enclosed in an electrical shield. The electrical shield may be electrically disconnected at the distal end of the bundle. The bundle has a proximal end. The electrical shield may only be grounded at the proximal end of the bundle.

Having described intrusive medical devices generally, attention is now directed to more detailed descriptions of embodiments of said intrusive medical devices.

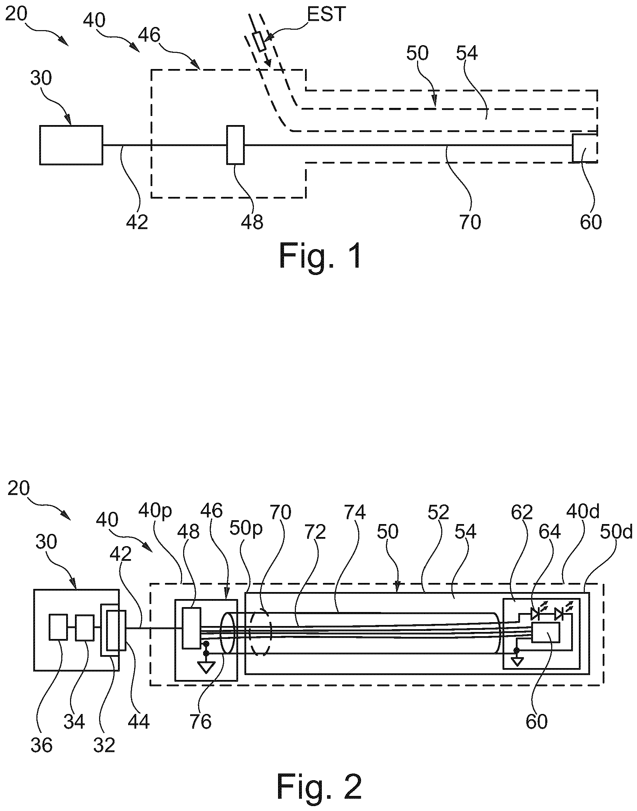

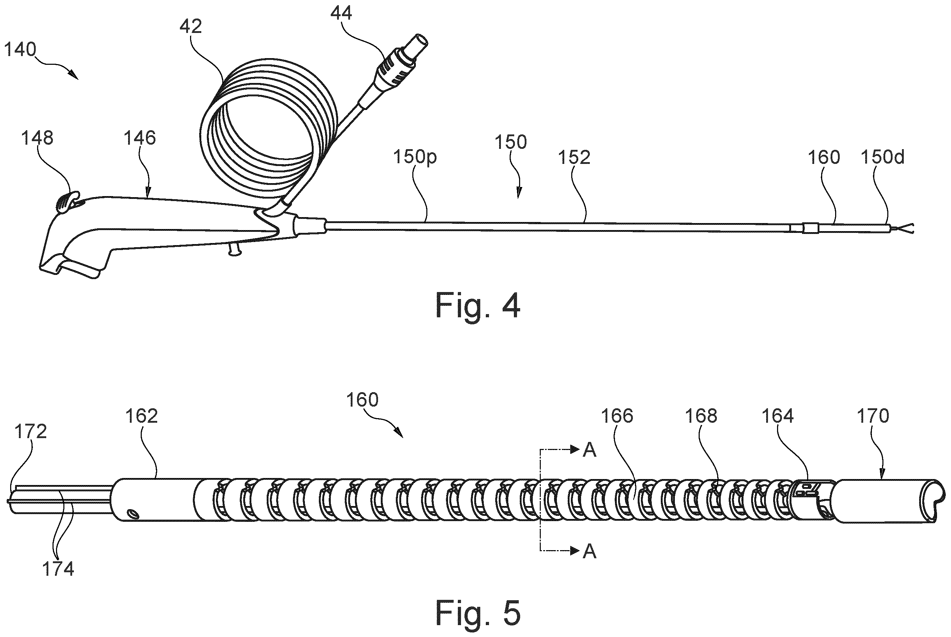

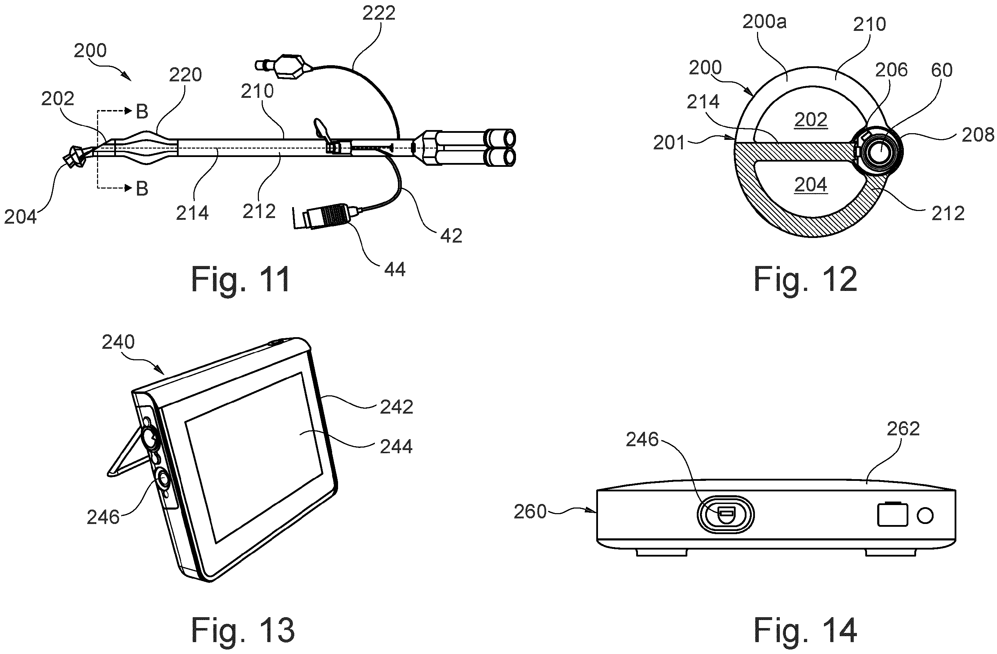

Figs. 1 and 2 are schematic diagrams of an embodiment of a visualization system 20 including a video processing apparatus 30 electrically connected to an intrusive medical device 40 by a cable 42 and a cable connector 44. Examples of the intrusive medical device 40 include endoscopes (described with reference to FIGS. 4 to 10), dual-lumen tubes (described with reference to FIGS. 11 and 12), and any other device configured for insertion into the body of a patient, human or animal, and containing a light source at a distal end thereof. An electrosurgical tool EST useable with the visualization system 20 is also shown. Some of the features of the intrusive medical device 40 shown in FIGS. 1 and 2 illustrate optional features and components. Embodiments of light sources include light emitting diodes (LEDs or OLEDs) and laser diodes. Laser diodes may under some conditions be advantageous as they can emit more light than LEDs or OLEDs, which could be used to provide the same light intensity from a smaller footprint of the light sources, or used to provide more light, which could positively influence image quality. On the other hand laser diodes emit coherrent and unidirectional light, which may be a disadvantage in some cases. At present LEDs are preferred.

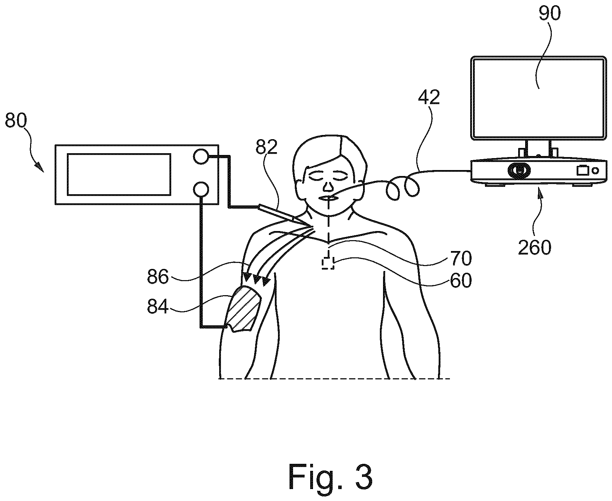

As shown in FIG. 2 and described in detail further below, the video processing apparatus 30 comprises a cable connector receptor 32, an input circuit 34, and a processor 36. Optionally, the VPA 30 may include a housing supporting a display screen connected to the processor 36 and operable to present images provided by the processor 36. A VPA with a display screen is described with reference to FIG. 13. A VPA without display screen is described with reference to FIGS. 3 and 14. The input circuit 34 may comprise a deserializer circuit to convert the data or signals provided by an image sensor of a camera from serial to parallel format. The processor 36 may comprise an FPGA, CPU, GPU, and combinations thereof. An FPGA may be programmed to conduct video processing to improve the images and a CPU may be provided to configure a graphical user interface (GUI) and superimpose the GUI onto the images. The combined content may be supplied to the FPGA, which may be connected to a video output circuit.

The intrusive medical device 40 has a proximal end 40p and a distal end 40d spaced apart from the proximal end 40p and comprises the cable 42 and the cable connector 44, an optional positioning interface 46, an optional circuit board 48, a tubular member 50 having a proximal end 50p, a distal end 50d, and a tubular member wall 52 defining a lumen 54 configured to receive therethrough the EST. The tubular member 50 extends from the proximal end 40p to the distal end 40d.

The intrusive medical device 40 also comprises a camera 60, an optional circuit board 62, light emitting diodes (LED) 64, a cable bundle 70 with conductors 72 and an electrical shield 74, and a proximal-end shield ground 76 (e.g. a connection of the electrical shield 74 to ground). The camera may include an image sensor, lenses and a lens support coupled with the image sensor. The image sensor may have a cross-section of less than 2.0 mm on each side, preferably less than 1.9 mm on each side. The cable bundle 70 extends from the distal end 50d to at least the proximal end 50p. The bundle 70 consisting of a first and a second coaxial cable within a common electrical shield 74, the cable bundle extending from the proximal end to the distal end and electrically connected to the camera and light source at the distal end; the first coaxial cable comprising a first cable central conductor, a first cable insulating layer, and a first cable shield; the second coaxial cable comprising a second cable central conductor, a second cable insulating layer, and a second cable shield; one of the first or second cable shield electrically connecting to the light source and configured to supply power; another of the first or second cable shield electrically connecting to the camera and configured to supply power; one of the first or second cable central conductor electrically connecting to the camera and configured to transfer a video signal; another of the first or second cable central conductor electrically connecting to the camera and configured to transfer a clock signal; and the common electrical shield electrically connecting camera and light source to ground.

As described with reference to FIGS. 1 and 2, the intrusive medical device may comprise a tubular member defining a lumen extending from the proximal end to the distal end. However, the intrusive medical device may be devoid of such a lumen. For example, some endoscopes are used for inspection and can be introduced into the patient through a lumen of another intrusive medical device that has one or more lumens. The advantages of the electrical noise mitigation features described herein are equally applicable to such an endoscope because electrosurgery may be conducted with different tools and systems. In some systems the EST is introduced through the lumen of the intrusive medical device 40. In some systems the EST is not introduced through the lumen of the intrusive medical device 40.

FIG. 3 illustrates an example of an electrosurgery system 80 comprising two electrodes, 82 and 84, that form an electrical path 86 via the patient's body. The EST, e.g. electrode 82, may be introduced into the patient through a lumen of a tubular member (not shown). A separate bundle 70 connected to the camera 60 and the cable 42 is shown to illustrate that the bundle 70 does not necessarily form part of an intrusive medical device having the tubular member through which the EST is introduced into the patient. The cable 42 is connected to a VPA 260, which is described with reference to FIG. 14 and includes a separable display screen 90 and a display screen support 92, which is configured, in normal use, to be detachable from the VPA 260 and the display screen 90. The display screen is operable to present images and video streams generated and transmitted by the camera 60. A VPA 240 (described below) may be used instead of the VPA 260.

FIGS. 4 and 5 show an embodiment of an intrusive medical device 40, exemplified by an endoscope 140, comprising a positional interface exemplified by a handle 146, which includes a steering control 148 operable to steer the distal end of the endoscope 140, as is known in the art, by alternatively pulling on steering wires 174, shown in FIG. 5, responsive to movement of the steering control 148. The endoscope 140 comprises an insertion cord 150 having a proximal end 150p and a distal end 150d, the insertion cord 150 including an insertion tube 152 and a bending section 160. A distal tip comprising a tip housing 170 extends from the bending section 160. The camera 60 is positioned in the tip housing 170. Alternatively the camera 60 may at least partly be positioned in the tip housing 170. The bending section 160 may comprise a single-piece polymeric structure comprising a plurality of segments 166 intermediate a proximal segment 162 and a distal segment 164 that is connected to the tip housing 170. The segments are interconnected by polymeric strips 168, or hinges, which form part of the one-piece structure and bend upon tensioning of the steering wires 174. A working channel tube 172, which is an example of the tubular member 50, provides the lumen 54 for introduction of the EST.

Instead of a single-piece polymeric structure, the bending section can also be assembled from multiple pieces. Such assemblies may be formed from two single-piece polymeric structures that elongate and form two, opposing, longitudinal halves of the finished bending section. Such assemblies may also be formed from individual segments assembled via hinges.

The endoscope 140 may be a single-use device. Single-use devices are designed to be low cost and disposable, not to be cleaned and sterilized after use and not to be used after cleaning.

A positioning interface functions to control the position of the insertion cord. A handle is an example of a positioning interface and, unless stated otherwise, the terms are used interchangeably. The handle also functions to provide the steering control, e.g. knobs, levers, buttons, and the like, to steer the field of view of the camera. Alternatively, a different positioning interface can be provided that is connected to the insertion cord and is detachably connected to a robotic arm. The insertion cord thus extends from the robotic arm, and the intrusive medical device is detachable from the robotic arm. The robotic arm responds to signals, including voice commands from an operator, to rotate, translate, and otherwise position the proximal end of the insertion cord, as an operator would do manually. The positioning interface can include control actuators, including manual control actuators. Alternatively or additionally, control actuators can be provided in or on the robotic arm or by the robotic system including the robotic arm, thereby potentially reducing the cost of the intrusive medical device. Example control actuators include single axis actuators, including linear motion actuators. A linear motion actuator may comprise a threaded rod coupled to a threaded nut portion, in which a motor rotates the rod to translate the nut portion.

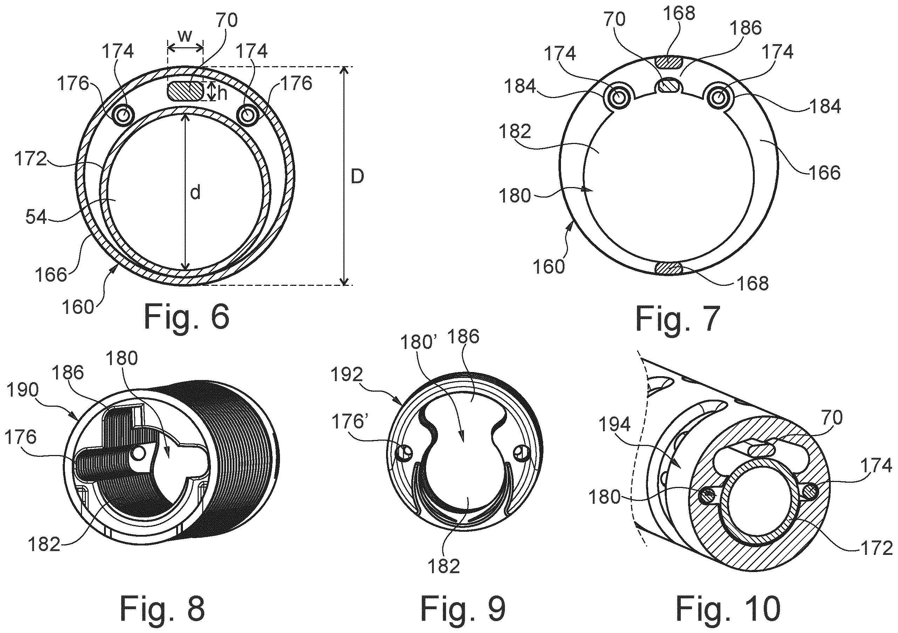

FIG. 6 is a schematic view of a cross-section A-A of the bending section 160 showing a cross-section of one of the segments, a cross-section of the working channel tube 172, the lumen 54, or working channel lumen, the steering wires 174, steering wire guide tubes 176, and the cable bundle 70. The bending section 160 has an outer diameter D. The steering wire guide tubes 176 surround the steering wires 174, which are connected to the tip housing 170 at one end and to the steering control 148 at the opposite end. A wall of the segment 166 (not shown in FIG. 6) comprises cut-outs or apertures through which the steering cable guide tubes 176 pass and cut-outs or apertures for the cable bundle 70. Examples of the cut-outs or apertures are shown and described with reference to FIGS. 7 to 9. The cable bundle 70 illustrated is non-circular with a flat or rectangular cross-section having a height h and a width w. This is advantageous as the cable bundle 70 can be arranged with the shortest dimension (i.e. the height) in radial direction of the cross-section, whereby the cable bundle 70 will take up a minimum percentage of the bending section outer diameter D enabling provision of a small outer diameter D or a larger inner diameter d of the lumen 54, both of which are desirable.

A sleeve or bending cover (not shown) may be provided over the bending section 160 to fluidly seal the spaces between adjacent segments 166. The outer diameter of a segment 166 may be substantially similar or the same as the outer diameter of the tip housing 170. In some embodiments the outer diameter is less than 4.2 mm, less than 3.6 mm, less than 3.4 mm, less than 3.2 mm, and even less than 3.0 mm with a minimum outer diameter of 1.8 mm.

In a variation A of the present embodiment, the wall thickness of the wall of the working channel tube is between, and including, 0.10 and 0.20 mm, preferably between, and including, 0.12 and 0.18 mm, and more preferably about 0.15 mm, and the external diameter of the working channel tube is between, and including, 2.0 and 3.0 mm, preferably between, and including, 2.20 and 2.80 mm, and more preferably between, and including, 2.40 and 2.60 mm.

In a variation B of the present embodiment, a minimum dimension of the of the cable bundle 70, is between, and including, 0.40 and 0.56 mm, preferably between, and including, 0.44 and 0.52 mm, and more preferably between, and including, 0.45 mm and 0.50 mm, and the external diameter of the working channel tube is between, and including, 2.0 and 3.0 mm, preferably between, and including, 2.20 and 2.80 mm, and more preferrably between, and including, 2.40 and 2.60 mm.

In a variation C of the present embodiment, an internal diameter of the insertion tube is less than 3.8 mm, or less than 3.4 mm, or less than 3.0 mm.

Variations of the present embodiment may be combined to form additional variations of the embodiment. Thus, variation A may be combined in a new variation with variation B, variation A may be combined in a new variation with variation C, variation A may be combined in a new variation with variations B and C, and variation B may be combined in a new variation with variation C.

FIGS. 7 to 10 provide examples of segment walls provided to maintain separation of the steering wires 174 and secure the bundle 70 in the bending section 160. FIG. 7, for example, shows a common opening 180 defining a working channel tube aperture 182, steering wire cut-outs 184 (cut out from the periphery of the working channel tube aperture 182), and a bundle cut-out 186. Providing the cut-outs around the working channel tube aperture 182 allows for reduction of the diameter and increased flexibility of the bending section 160. FIG. 8 shows another example of the opening 180 in a bending section 190. The opening 180 in this example includes, as in FIG. 7, three cut-outs for the steering wires 174 and the bundle 70. Additional tubes may be provided in the cut-out 186 due to its relatively larger cross-sectional area relative to the cross-section of the bundle 70. FIG. 9 shows an example of a common opening 180' in a bending section 192. The opening 180' in this example includes, as in FIG. 7, a cut-out for the the bundle 70, and perhaps for other tubes or components, and apertures 176' for steering wires 174. Additional tubes may be provided in the cut-out 186 due to its larger, relative to the cross-section of the bundle 70, cross-sectional area. FIG. 10 shows an example of the common opening 180 in a bending section 194. The bending section 194 differs from the bending section 192 in that cut-outs are provided for the steering wires 174 instead of openings. As used herein, common opening refers to an opening that accomodates a working channel tube and one or more of steering wires and a bundle. As shown, accomodation of the steering wires and bundle is provided by cut-outs. Openings for the working channel tube, steering wires and the bundle can also be provided, size permitting, via individual openings instead of a common opening.

The camera 60 may have a cross-section of less than 2.0 mm on each side. The outside diameter of the tip housing 170 may be about 3.0 mm, and preferrably about 2.8 mm, and more preferrably 2.8 mm or less. The term "about" is intended to define a range of +/- 10% from the specified numeral.

As the dimensions of the camera and the bending section are continuously reduced for the benefit of the patient, the size of the wires and cables increase as a percentage of the cross-section. It is valuable, to continue reductions in size and cost, to identify cable configurations, the cables comprising the wires, shielding, jacket, etc., that use smaller wires while still avoid the negative effects of electrical noise. The success of the mitigation effort is dependent on the image sensor and deserializer used, the length of the bundle, and the structure surrounding it.

FIGS. 11 and 12 show another embodiment of an intrusive medical device 40, exemplified by a dual-lumen tube 200. FIG. 12 shows a cross-section B-B of the tube 200. The dual-lumen tube 200 comprises a tubular member 200a having a peripheral (or circumferential) wall 201 defining a first lumen 202 and an illumination lumen 208 therein. The camera 60 and LEDs 206 are positioned in the illumination lumen 208 located in the wall of the tube 200.

The dual-lumen tube can be an endobronchial or an endotracheal tube, for example. The dual-lumen tube 200 may also have a second lumen 204. The peripheral wall 201 may consist of a first portion 210 and a second portion 212 divided by a medial wall 214. An inflatable cuff 220 is provided at the distal end and is connected via an inflation lumen (not shown) to an inflation tube 222 that can be connected to a pump to inflate the cuff 220. As with the endoscope 140, it is desirable to reduce the size of the device and increase its flexibility, for example by reducing the wall thickness of the tube, which requires reductions in the size of the camera 60 and the bundle 70. Although not shown, the bundle 70 is positioned in the illumination lumen 208 and communicativelly connects the camera 60 with the cable connector 44. A single-lumen tube can also include the features described herein, including the first lumen 202, the camera 60, the LEDs 206, and the bundle 70, positioned in the illumination lumen 208 located in a wall of the tube 200.

A VPA 240 with a display screen 244 is shown in FIG. 13. The VPA 240 includes a housing 242 and one or more cable connector receptor 246 configured to receive the cable connector 244. The housing 242 supports and is assembled in one-piece with the display screen 244, referred to as an "integral" display screen, by contrast with a "separable" display screen. The terms "integral" and "separable" reflect a device in its assembled form, as it is in use, by contrast with a device in its unassembled or disassembled form. A VPA 260 without a display screen 244 is shown in FIG. 14 and was previously shown in FIG. 3 with a separable display screen 90. The VPA 260 includes a housing 262 and one or more cable connector receptacles 246 configured to receive the cable connector 44. In both the VPA 240 and the VPA 260, a separable display screen can be communicativelly connected thereto, as is known in the art, via an ethernet, wireless, AVI, HDMI, or other data interfaces. Upon connection of an intrusive medical device 40, the VPA 240 or 260 present images or video streams on the integral and/or the separable display screen, as is known in the art.

In the foregoing description intrusive medical devices were described, the intrusive medical devices comprising: a proximal end and a distal end spaced apart from the proximal end; a camera positioned at the distal end; and a bundle consisting of two coaxial cables with a common shield, the bundle extending from the proximal end to the distal end and electrically connected to the camera at the distal end. In some embodiments, but not others, the intrusive medical devices include a tubular member defining a lumen therein, the tubular member extending from the proximal end to the distal end of the device. The two coaxial cables comprise five conductors in total including a ground conductor, a camera power conductor, illumination power conductor, a clock conductor and a video signal conductor. Embodiments of the bundle 70 are described below.

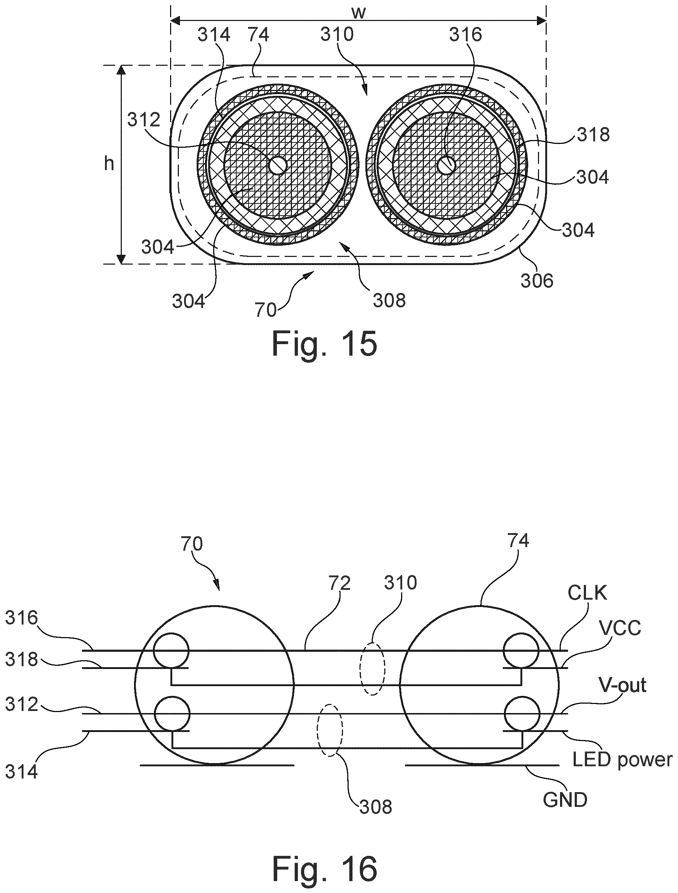

Attention is now directed to an embodiment of the bundle 70, shown in FIG. 15. The bundle 70 comprises first coax cable 308 and a second coax cable 310 arranged within a common shield 74. The first coax cable 308 comprises a central conductor 312, an insulator 304 and a shield 314. The insulator may for example be perfluoroalkoxy. The central conductor 312 may be a wire or a stranded wire comprising two or more strands, such as seven strands. The shield of the first coax cable 308 may be formed of a braiding of wires or a spiral wire around the insulator. The shield 314 is electrically conductive and constitutes a conductor 72. Preferably the shield is not formed of an aluminium foil, as the electrical resistance of such aluminium foil is considered too high to efficiently constitute a conductor in the present disclosure. Similarly the second coax cable 310 comprises a central conductor 316, an insulator 304 and a shield 318. The central conductor may be a wire or a stranded wire comprising two or more strands. The shield 318 of the second coax cable 310 may be formed of a braiding of wires or a spiral wire around the insulator. The shield 318 is electrically conductive and constitutes a conductor 72. Preferably the shield is not formed of an aluminium foil, as the electrical resistance of such aluminium foil is considered too high to efficiently constitute a conductor in the present disclosure. The coax cables may have an insulator 304 arranged on the outer surface of the shield. The insulator may be a lacquer, which enables very compact cable bundles, but such lacquers are generally fragile and may deteriorate during assembly or use of the intrusive medical device. It is hence at present considered a better option to provide a coating of e.g. perfluoroalkoxy (PFA), Polyethylene (PE) or Polypropylene (PP) on the coax cables. The first and second coax cable may be identical, which would provide simplicity and potentially provide a low cost, but may differ, e.g. if considered to be beneficial in view of increased shielding of one or the other conductor, or if one or more conductors are subject to concerns on electrical resistance. The common shield 74 may be formed of a braiding of wires, a spiral wire or a wrap or foil, such as a tinned copper wrap. The common shield 74 is electrically conductive and electrically connects to ground. The common shield 74 may comprise two layers, such as two braidings on top of each other for increased strength and robustnes of the cable bundle, which may be advantageous for handling during manufacturing. The common shield may be provided with an insulator, such as a jacket 306 of insulating material. The insulator might be a protective sleeve, or jacket, 306 that provides strength to the bundle, permitting use of smaller gauge wires. Alternetively the insulator may be a lacquer, thereby enabling a very compact cable bundle, however with an increased risk of breakage of the wires or deterioration of the lacquer covering in production or during use, all of which could lead to loss of live image or negatively influence the image quality. The wires are selected based on their current carrying capacity and physical strength and therefore may be larger or smaller depending on their function. To reduce size and increase flexibility of the tubular member, it is desirable to use the smallest wire that can perform the selected function. Therefore, one of the wires might be larger than another of the wires. The resistance of the wire and hence voltage drop across the length of a wire is another limiting factor in some functions. For example, too much voltage drop, due to too small wire diameter, can cause image degradation in the video signal conductor of the bundle. The inventors have found that particular combinations of wires, shields, and conductor positions, result in surprisingly good electrical noise mitigation, for example, but not necessarily exclusively, of cross-talk between the clock onto the video signal.

The cable bundle 70 as depicted in FIG. 15 is operable with image sensors comprising four connection pads. Such image sensors and pad arrangements have been developed to reduce the size of the image sensor. The bundle 70 consisting of two coax cables 308, 310 within a common shield 74 enables a very compact cable bundle construction with a flat configuration having a height h approximately half of the width w. A flat configuration of the cable bundle 70 may be an advantage in some endoscope constructions as the low height h means that the cable bundle 70 may be incorporated in the endoscope with very little influence on the diameter thereof. Reduction in bundle profile is thus beneficial to reduce the cross-section area of the intrusive medical device 40 and benefit from use of smaller cameras.

FIG. 16 is a schematic diagram of the cable bundle 70 of the embodiment of FIG. 15 according to a variant A. The first coax cable 308 comprises two conductors 72 as explained above, namely the central conductor 312 which in this variant electrically connects to the camera and carries the video signal conductor V-out, whereas the shield 314 electrically connects to the light source and is configured to supply power to the light sorce. The second coax 310 comprises two conductors as explained above. The central conductor 316 electrically connects to the camera and is configured to transfer the video clock signal CLK, whereas the shield 318 electrically connects to the camera and is configured to supply power to the camera VCC. The common shield 74 electrically connects both camera and LED to ground GND. With this set-up there is a high mitigation of cross-talk between clock signal and the video signal in that both signals are protected by shields. Further there is high mitigation of HF noise, e.g. from electrosurgical tools, as the signals are protected by the common shield 74 and the individual shields of the two coax cables 308, 310.

A variant B is illustrated in the schematic diagram of the cable bundle 70 of the embodiment of FIG. 15. The first coax cable 308 comprises two conductors as explained above, namely the central conductor 312 which in this variant electrically connects to the camera and is configured to transfer the video clock signal CLK, and shield 314, which here electrically connects to the light source and is configured to supply power LED power. The central conductor 316 of the second coax 310 electrically connects to the camera and is configured to transfer the video signal V-out, whereas the shield 318 electrically connects to the camera and is configured to supply power to the camera VCC. The common shield 74 electrically connects ground GND to both camera and LED. With this set-up there is also a high mitigation of cross-talk between clock signal and the video signal in that both signals are protected by shields. Further there is high mitigation of HF noise, e.g. from electrosurgical tools, as the signals are protected by the common shield 74 and the individual shields of the two coax cables 308, 310. Variant B may be advantageous if illumination intensity is changed by increasing or decreasing LED power, especially if illumination intensity is managed by pulsating LED power. Changing LED power, and especially pulsating LED power, could introduce electrical noise on the central conductor. The video signal V-out is generally considered more sensitive to electrical noise than the clock signal CLK, so it is considered advantageous to provide the LED power and the video signal V-out on different coax cables.

The central conductor of the coax cables should be as small as possible to provide a low profile of the cable taking due account the resistance of the conductor, which increases with decreasing cross-section of the conductor. The central conductor may be a single wire or a two or more strands making up the wire. Wire sizes of the central conductor in the range of 40 to 44 American Wire Gauge (AWG) are generally considered a suitable compromise for the embodiment, but may be larger, e.g. a 38 AWG wire, or smaller, e.g. a 46 AWG, a 48 AWG or even a 50 AWG wire for other embodiments. 40 AWG corresponds to an outer diameter of the wire of 0.079 mm for a single wire, whereas 44 AWG corresponds to an outer diameter of the wire of 0.051 mm for a single wire, and conductor resistance of e.g. 3.8 Ohm/m and 9.1 Ohm/m, respectively. Diameter of the center conductor is larger if is a multiple strand type. Conductor resistance also depends on the conductor material (e.g. copper or special alloys), whether it is a single wire or made up of strands, and whether the wire or strands of the wire are additionally for example tinned or silver plated. Outer diameter of the coax cable also depends on the thickness of the insulation etc. Examples of outer diameter of the coax cable is 0.33-0.37 mm for a 40 AWG 7-wire center conductor, and 0.22-0.24 mm for a 46 AWG 7-wire center conductor. Stranded wires are more flexible, but takes up a more space than single wires.

In an example of variation B, the first coax cable 308 is larger than the second coax cable 310. In one example, the first coax cable 308 is at least a 42 American Wire Gauge (AWG) wire and may be larger, e.g. a 40 AWG wire, whereas the second coax cable 310 is at most a 44 AWG wire, preferrably a 46 AWG wire. Wire gauge may also be based on the length of the cable. If the image sensor cable (inside the device) is 300 mm or less a thinner gauge may be used than if the cable is longer.

In a further variation, C, of the present embodiment, the conductors comprise the same type and size of conductor.

Variations of the present embodiment may be combined to form additional variations of the embodiment. Thus, variations A and B may be combined in new variations with variation C.

In a variation a single conductor is used as a shared power line to LED and camera, thereby avoiding to have separate power conductors for camera and LED. However, the power needed for camera and LED are often not the same, so a shared power line would require the provision of a voltage regulator or other power division means to divide the power provided by a shared power line to the camera and LEDs. Avoidance of power division means at the distal end of the intrusive medical device also enables reduction of the size of the intrusive portion, e.g. the distal end, of the intrusive medical device. A separate power conductor for the LEDs also facilitates potentially regulating the power up and down, or pulsating power, for adjusting the illumination, which may have a positive effect on image quality.

Separation of the video signal and clock conductors has resulted in sufficient electrical noise mitigation from cross-talk from the clock signal. The video signal conductor may be used to transfer an analog out signal from the camera and control signals between the camera and the VPA. The analog out signal includes the video frames and images. The control signals may be provided via a serial peripheral interface (SPI) and multiplexed with the analog signals and may include gain and exposure camera settings.

The electrical shield may be electrically disconnected at the distal end of the bundle. The bundle has a proximal end. The electrical shield may only be grounded at the proximal end of the bundle. The electrical shield may be comprised by a braid or spiral wire. Other types of electrical shields may be used as well.

The uninsulated wires range in size (e.g. diameter) from 36 AWG to 50 AWG. As is well known, AWG denotes the American Wire Gauge standard. Dimensions of the wires are given in ASTM standard B 258 (ASTM B258-18). The AWG tables are for a single, solid and round conductor. The AWG of a stranded wire is determined by the cross-sectional area of the equivalent solid conductor. Because there are also small gaps between the strands, a stranded wire will always have a slightly larger overall diameter than a solid wire with the same AWG. 36 AWG wire, for example, has an outer diameter of 0.127 mm. 32 AWG wire has an outer diameter of 0.202 mm. 40 AWG wire has an outer diameter of 0.080 mm. 42 AWG wire has an outer diameter of 0.063 mm. 44 AWG wire has an outer diameter of 0.050 mm. 46 AWG wire has an outer diameter of 0.040 mm. In one example, the 40 AWG wire insulation thickness is 0.040 mm, a shield thickness (braid) is 0.020 mm, an outer insulation on the shield is 0.030 mm, an outer shield is 0.060 mm (double braiding), and an outer jacket is 0.080 mm, bringing the total outer width of the cable bundle to 0.800 mm and the height to 0.540 mm.

Generally, all the wires are at most 38 AWG wires in size, and may be 38, 40, 42, 44, 46, 48 or 50 AWG wires. Smaller gauge wires can aid in reducing the cross-sectional size of the intrusive portion of the intrusive medical device and increasing bending flexibility but at the expense of noise sensitivity. When size reduction is not needed heavier gauge wires may be preferrable.

A test was performed to check for sensibility of an intrusive medical device to electrical noise generated by an electrosurgical tool. The test was performed to determine high frequency (HF) immunity performance of a dual coax shielded cable 44 AWG with a length of 1 m. The electrosurgical tool used was an ERBE VIO^{®} 3 with APC3 module and an FIAPC probe 2200 A, Ø 3.2 mm, length 2.2 m (5 kVp). Excellent performance of the cable was found with no disturbing interference, so image quality was good.

Previous tests on other wire types revealed problems with interference. For example, tests were performed on single wires (non-coaxial). Problems are found to relate not only to interference on the video signal, but also to interference with the camera power. In the test the camera supply was 3.42V DC, but interference was found to influence the voltage measured at the camera in the tip, so the voltage measured range from 1.88 V to 4.98 V. The camera requires minimum 3.14 V for normal operation, and even though the low of 1.88 V was only measured for a short period of time (~4 ns), it was enough to trigger the camera to reboot. This reset and restart of the camera gives rise to flicker on the image.

Although some embodiments have been described and shown in detail, the invention is not restricted to them but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same hardware components. The mere fact that certain measures are recited in mutually different dependent items or described in different embodiments does not indicate that a combination of these measures cannot be used to advantage.

The terms "first," "second," and the like in the description and in the claims, if any, are used for distinguishing between similar elements and not necessarily for describing a particular sequential or chronological order. It is to be understood that any terms so used are interchangeable under appropriate circumstances such that the embodiments described herein are, for example, capable of operation in sequences other than those illustrated or otherwise described herein.

It should be emphasized that the term "comprises/comprising" are generally interpreted to be open ended terms which specify the presence of stated features, integers, steps or components but do not preclude the presence or addition of one or more other features, integers, steps, components or groups thereof. The terms "consisting of" or "consists of" are closed terms, and include only the components, structures, steps, or the like specifically listed in conjunction with such terms, as well as that which is in accordance with U.S. Patent law.

### Reference numerals:

| REFERENCE NUMERALS: | |
|---|---|
| 20 | Visualization system |
| 30, 240, 260 | Video processing apparatus (VPA) |
| 32 | Cable connector receptor |
| 34 | Input circuit |
| 36 | Processor |
| 40 | Intrusive medical device |
| 40p | Proximal end |
| 40d | Distal end |
| 42 | Cable |
| 44 | Cable connector |
| 46 | Positioning interface |
| 48 | Circuit board |
| 50 | Tubular member |
| 50p | Proximal end |
| 50d | Distal end |
| 52 | Tubular member wall |
| 54 | Lumen |
| 56 | Proximal end |
| 58 | Distal end |
| 60 | Camera |
| 62 | Circuit board |
| 64 | Light emitting diode (LED) |
| 70 | Cable bundle |
| 72 | Conductor |
| 74 | Electrical shield |
| 76 | Proximal-end shield ground |
| 80 | Electrosurgery system |
| 82 | Electrode |
| 84 | Electrode |
| 86 | Electrical path |
| 90 | Display screen |
| 92 | Display screen support |
| 140 | Endoscope |
| 146 | Handle |
| 148 | Steering control |
| 150 | Insertion cord |
| 150d | Distal end |
| 150p | Proximal end |
| 152 | Insertion tube |
| 160, 190, 192, 194 | Bending section |
| 162 | Proximal segment |
| 164 | Distal segment |
| 166 | Segment |
| 168 | Hinge |
| 170 | Tip housing |
| 172 | Working channel tube |
| 174 | Steering cable |
| 176 | Steering cable guide tubes |
| 176' | Apertures |
| 180 | Common opening |
| 180' | Common opening |
| 182 | Working channel tube opening |
| 184 | Steering cable cut-out |
| 186 | Bundle cut-out |
| 192 | Bending section |
| 194 | Bending section |
| 200 | Dual-lumen tube |
| 200a | Tubular member |
| 201 | Circumferential wall |
| 202 | First lumen |
| 204 | Second lumen |
| 206 | LED |
| 208 | Illumination lumen |
| 210 | First portion of wall |
| 212 | Second portion of wall |
| 214 | Medial wall |
| 220 | Cuff |
| 222 | Inflation tube |
| 242, 262 | Housing |
| 244 | Display screen |
| 246 | Cable connector receptor/receptacle |
| 304 | Insulator/insulation |
| 306 | Jacket |
| 308 | 1^{st} Coax cable |
| 310 | 2^{nd} coax cable |
| 312 | Central conductor, 1^{st} coax cable |
| 314 | Shield, 1^{st} coax cable |
| 316 | Central conductor, 2^{nd} coax cable |
| 318 | Shield, 2^{nd} coax cable |
| d | Diameter of inner lumen of working channel tube |
| D | Outer diameter of bending section |
| EST | Electro-surgical tool |
| H | Height of cable bundle |
| W | Width of cable bundle |

## Claims

1. An intrusive medical device comprising:
a proximal end (40p) and a distal end (40d) spaced apart from the proximal end;
a tubular member (50) defining a lumen (54) therein, the tubular member extending from the proximal end to the distal end;
a camera (60) and a light source positioned at the distal end; and
a cable bundle (70) consisting of a first coaxial cable (308) and a second coaxial (310) cable within a common electrical shield (74), the cable bundle extending from the proximal end to the distal end and electrically connected to the camera and light source at the distal end;
the first coaxial cable (308) comprising a first cable central conductor (312), a first cable insulating layer, and a first cable shield (314);
the second coaxial cable (310) comprising a second cable central conductor (316), a second cable insulating layer, and a second cable shield (318);
one of the first or second cable shield electrically connecting the light source and configured to supply power;
the other of the first or second cable shield electrically connecting the camera and configured to supply power;
one of the first or second cable central conductor electrically connecting the camera and configured to transfer a video signal;
the other of the first or second cable central conductor electrically connecting the camera and configured to transfer a clock signal; and
the common electrical shield (74) electrically connecting ground to camera and light source.

2. The intrusive medical device of claim 1, wherein the coaxial cables are untwisted.

3. The intrusive medical device of claim 1 or 2, the first cable shield electrically connecting the light source and configured to supply power; the first cable central conductor electrically connecting the camera and configured to transfer the video signal; the second cable shield electrically connecting the camera and configured to the supply power; the second cable central conductor electrically connecting the camera and configured to transfer the clock signal.

4. The intrusive medical device of claim 1 or 2, the first cable shield electrically connecting the light source and configured to supply power; the first cable central conductor electrically connecting the camera and configured to transfer the clock signal; the second cable shield electrically connecting camera and configured to supply power; the second cable central conductor electrically connecting the camera and configured to transfer the video signal.

5. The intrusive medical device of any one of claims 1-4, the coaxial cables being micro-coaxial cables with a center conductor maximum cross-sectional area in the interval of 0.0005 mm² to 0.0050 mm², such as in the interval of 0.0008 mm² to 0.0032 mm², such as 0.0012 mm².

6. The intrusive medical device of any one of claims 1-5, wherein the cable bundle has a height in the interval of 0.1 to 0.7 mm, such as in the interval of 0.2 mm to 0.6 mm, such as 0.35 mm.

7. The intrusive medical device of any one of the claims 1-6, wherein the cable bundle has a width in the interval of 0.2 mm to 0.9 mm, such as in the interval of 0.4 mm to 0.75 mm, such as 0.60 mm.

8. The intrusive medical device of any one of the claims 6-7, wherein the cable bundle has a ratio of height to width in the interval of 0.55 to 0.75, such a 0.67.

9. The intrusive medical device of any of the preceding claims, wherein the intrusive medical device comprises an endoscope including:
the proximal end comprising a positioning interface (146) having a distal end;
an insertion cord (150) connected to and extending from the distal end of the positioning interface and comprising an insertion tube (152), a bending section (160), and a tip housing (170), the camera positioned in the tip housing, the tubular member extending from the positioning interface through the insertion tube to the tip housing,
wherein the bundle extends from the positioning interface to the tip housing.

10. The intrusive medical device of claim 8, wherein the bending section comprises a diameter in the interval of 1.8 mm to 6.0 mm, such as in the interval of 2.0 mm to 3 mm, such as 2.5 mm.

11. The intrusive medical device of claims 5 and 10, wherein a ratio of the cable bundle height to bending section diameter is less than 0.2, such as in the interval of 0.04 to 0.16, e.g. approximately 0.1.

12. A visualization system (20) comprising:
the intrusive medical device (40, 140, 200) of any one of the preceding claims; and
a video processing apparatus (30) configured to communicatively connect with the intrusive medical device to receive a video stream therefrom.
